# EUROPEAN PATENT SPECIFICATION

(11) **EP 1 763 600 B1**
(45) Date of publication and mention of the grant of the patent: **30.03.2011**
(21) Application number: 05766931.9
(22) Date of filing: 23.06.2005
(51) Int. Cl.: D02G 3/12, A61F 2/06, A61L 27/00, A61L 31/00, A61L 31/12

(54) **METALLIC FIBERS REINFORCED TEXTILE PROSTHESIS**
MIT METALLDRÄHTEN VERSTÄRKTE TEXTILPROTHESE
PROTHESE TEXTILE RENFORCEE PAR DES FIBRES METALLIQUES

(30) Priority: 24.06.2004 US 876212
(43) Date of publication of application: 21.03.2007
(73) Proprietor: Boston Scientific Scimed, Inc., Maple Grove, MN 55311-1566 (US)
(72) Inventor: DONG, Jerry (Qing), Oakland, NJ 07436 (US)
(74) Representative: Vossius & Partner
(86) International application number: PCT/US2005/023057
(87) International publication number: WO 2006/002439

(56) References cited:
- EP-A- 0 334 024
- EP-A- 1 312 705
- US-A- 3 582 444
- US-A- 4 813 219
- US-A1- 2001 047 198
- US-B1- 6 176 875
- US-B1- 6 689 162

## Description

### FIELD OF INVENTION:

The present invention is directed to an inventive yarn and prostheses therefrom. Additionally, a method for making the inventive yarn and prosthesis is also contemplated.

### BACKGROUND OF RELATED TECHNOLOGY:

An intraluminal prosthesis is a medical device used in the treatment of diseased blood vessels. An intraluminal prosthesis is typically used to repair, replace, or otherwise correct a diseased or damaged blood vessel. An artery or vein may be diseased in a variety of different ways. The prosthesis may therefore be used to prevent or treat a wide variety of defects such as stenosis of the vessel, thrombosis, occlusion or an aneurysm.

One type of intraluminal prosthesis used in the repair of diseases in various body vessels is a graft. A graft is a commonly known type of intraluminal prosthesis which is used to repair and replace various body vessels. A graft provides a lumen through which blood may flow. Moreover, a graft is often configured to have porosity to permit the ingrowth of cells for stabilization of an implanted graft while also being generally impermeable to blood to inhibit substantial leakage of blood therethrough. Grafts are typically tubular devices which may be formed of a variety of materials, including textile and non-textile materials.

Grafts are typically flexible to provide compliance within a bodily lumen or within the bodily system. Such flexibility may result from the stretching of the textile yarns forming the graft. Such stretching, however, may effect the securement of the graft to the bodily lumen, which is typically secured by the use of sutures, as well as the dilation of the graft. Graft dilation is a common phenomenon following AAA repair. It is more pronounced in knitted grafts than in woven grafts. The knitted grafts can dilate as much as about 43% and woven grafts can dilate to about 26%. Graft dilation is affected by the textile structure, processing method and elongation of the yarn. Textile yarn can have a breaking point below the range of the dilation, such as with DuPont Dacron PET yarn which has a breaking elongation in a range of 30-50%. In other words, the graft flexibility may create undesirable stresses on the textile structure of the implanted graft and material failure.

Grafts are deployed in a vascular system by means of a catheter delivery system which passes the graft through the lumen of the blood vessel for deployment at the desired location. These standard delivery systems for delivering such prostheses intraluminally generally include catheters with the prosthesis removably mounted to the distal end of the catheter. Quite often a catheter, introducer sheath, or other similar retaining means, is disposed over the prosthesis to removably support the prosthesis on the catheter. Once the prosthesis is situated in the target site in the lumen, the catheter is removed by pulling the sheath or retaining means away from the prosthesis to allow the expansion.

Since catheter delivery is typically done under a fluoroscope or other similar x-ray type viewing mechanism, the movement of traditional textile vascular grafts during deployment cannot be fluoroscopically viewed. Further, as with traditional surgically implanted grafts, catheter implanted grafts must be longitudinally flexible to conform to the shape of the vessel which it is repairing. Also, such grafts should be capable of a certain degree of longitudinal expansion to conform to the length of the blood vessel which is to be replaced. Finally, the graft, once implanted by the catheter delivery system, must readily return to its open tubular shape and maintain that shape during use. This is particularly important where the graft is implanted by a catheter as the graft must be tightly compressed and packed so as to fit within the hollow lumen of the catheter.

Thus, there is a need for a prosthesis to optimize elasticity, flexibility, and radial expansion. Further, there is a need for a prosthesis having increased mechanical strength, abrasion resistance and a certain degree of radial structural integrity. Furthemore, there is a need for a prosthesis having radiopacity.
A graft with polymer coated metal filaments is disclosed in US 6689162 B1.

### SUMMARY OF THE INVENTION:

The present invention seeks to overcome the deficiencies of the currently available prostheses. More specifically, the present invention provides a fabric and a prostheses with improved mechanical properties including a metallic component according to claim 1.

One aspect of the present invention includes an implantable prosthesis including a biocompatible implantable fabric having a textile construction of metal fibers.

Another aspect of the present invention includes an implantable prosthesis including a biocompatible implantable fabric having a textile construction. The textile construction including a composite yarn and metal fibers. The prosthesis is a graft.

A further aspect of the present invention includes a composite yarn is a biocompatible, implantable yarn having a metallic component and a non-metallic component combined together by a twisting, co-spinning, wrapping and combinations thereof. The total denier of the yarn is about 20 to about 300.

Another aspect the present invention includes a implantable prosthesis including a biocompatible implantable fabric having a textile construction including a composite yarn. The composite yarn includes a combination of a metallic component and a non-metallic component. The components are combined by twisting, co-spinning, wrapping and combinations thereof.

A further aspect of the present invention includes a variation of the twisted, co-spun, and/or wrapped techniques using multiple strands, and at least one strand is a metallic component.

An additional aspect of the present invention includes an implantable prosthesis including a biocompatible fabric having a textile construction including a composite yarn. The composite yarn includes a co-spun yarn of a stainless steel fiber and a polymer. The stainless steel is present in amounts of about 5% to about 100% of the total composite yarn. The composite yarn has a total denier of about 20 to about 300.

Yet a further aspect of the invention includes a method for making a implantable prosthesis including the steps of providing a composite yarn including a combination of a metallic component and a non-metallic component. The composite yarn being a combined by twisting, co-spinning, and combinations thereof. Forming the composite yarn into the implantable prosthesis using a selected textile construction. Various textile constructions include weaving, knitting, braiding, non-woven spinning and combinations thereof.

Further details regarding the present invention and variations thereof are further provided.

### BRIEF DESCRIPTION OF THE FIGURES

FIG. 1 is a schematic of a composite yarn of the present invention having a "S-twist" fornication;
FIG. 2 is a schematic of a composite yarn of the present invention having a "Z-twist" formation;
FIG. 3 is a schematic of a composite yarn of the present invention having a core yarn wrapped with a cover yam;
FIG. 4 is a schematic of a composite yarn of the present invention having a core yarn wrapped with multiple cover yarns;
FIG. 5 is a schematic of a composite yarn of the present invention having a core yarn wrapped with multiple cover yarns;
FIG. 6 is a schematic of a composite yarn having multiple core yarns wrapped with a cover yarn;
FIG. 6A is a schematic of a composite yarn having multiple core yarns wrapped with multiple cover yarns;
FIG. 7 is a perspective view of a woven tubular textile composite prosthesis of the present invention;
FIG. 7A is a perspective view of a woven patch textile composite prosthesis of the present invention;
FIG. 7B is an expanded view of a woven portion of the textile composite prosthesis of the present invention;
FIG. 8 is a perspective view of a braided tubular textile composite prosthesis of the present invention;
FIG. 8A is a expanded view of a diamond braid portion of the textile composite prosthesis of the present invention;
FIG. 8B is a expanded view of a regular braid portion of the textile composite prosthesis of the present invention;
FIG. 8C is a expanded view of a hercules braid portion of the textile composite prosthesis of the present invention;
FIG. 9 is a cross-sectional expanded view of a portion of a multi-layered interlocked three-dimensional braided textile composite prosthesis formed in accordance with an embodiment of the present invention;
FIG. 10 is a perspective view of a knitted textile composite prosthesis useful in the present invention;
FIG. 10A is an expanded view of a knitted portion of the textile composite prosthesis of the present invention;
FIG. 11 is an expanded view of a knitted portion having a two-needle underlap of the textile composite prosthesis of the present invention; and
FIG. 12 is an expanded view of a knitted portion having a three-needle underlap of the textile composite prosthesis of the present invention.

### DETAILED DESCRIPTION OF THE INVENTION

The present invention seeks to solve the deficiencies of the prior art by making prostheses having improved mechanical properties with a textile fabric made from a metallic component. A fabric or yarn made from the metallic component of the present invention provides a fabric having increased the durability of prostheses, thereby reducing the need for physicians to routinely remove, repair and replace vascular prostheses that have been implanted.

Further, the metallic component of the present invention provides a radiopaque guideline or marker for viewing the implanted prosthesis fluoroscopically. Radiopaque markers assist the surgeon to visualize the prosthesis both during and after implantation. The marker helps show the surgeon that the prosthesis is properly positioned. Also, it will indicate whether the prosthesis has dilated or collapsed after implantation.

Furthermore, the metallic component of the present invention provides strength, radial structural integrity, and abrasion resistance for the prosthesis or yarn made therefrom. Additionally, the metallic component provides controlled expansion and dilation of the prosthesis made therefrom. The properties of the metallic component provide the controlled expansion because of the rigidity and substantially inelasticity of the metallic properties.

The term "yarn" as used herein refers to a continuous strands, filaments, or materials in a form suitable for knitting, weaving, braiding, twisting, spinning, or otherwise intertwining to form a textile fabric. Yarn can occur in a variety of forms to include a spun yarn including staple fibers and strands usually bound together by twist, or multifilament yarn including many continuous filaments or strands. "Yarn" can also include manufactured fibers produced by, among other things, extrusion processes.

The term "component" herein refers to individual fibers, monofilament, multifilament, strands and materials in a form suitable for twisting, spinning, wrapping and otherwise intertwining to form the yarn of the present invention. Additionally, individual filaments which can make up the yarn may have any one of a variety of cross sections to include round, rectangular, serrated, bean-shaped or others. The individual components can be pre-twisted or spun prior to combining them to form the yarn of the present invention.

### INVENTIVE YARN

One embodiment of the present invention is a yarn including a metallic component. The metallic component is constructed of any biocompatible metallic material as known in the art such as stainless steel, titanium, nitinol, and the like. The metallic component should have a total diameter of from about 0.05mm to about 0.5mm, and preferably from about 0.18mm to about 0.38mm. The metallic component may include monofilament and multiple metallic filaments, with the total diameters of the filaments being within these ranges.

Another embodiment of the present invention is a composite yarn including a combination of non-metallic component and metallic component. The composite yarn of the present invention provides the benefits of the non-metallic component being elasticity, flexibility and radial expansion as well as the benefits of the metallic component being strength, radial structural integrity, controlled expansion and abrasion resistance.

The non-metallic component of the composite yarn of the present invention is biocompatible. The non-metallic component may be constructed from one of a variety of available natural and man-made fibers. These materials include polyester, PTFE, polypropylene and combinations thereof. The man-made fibers in this group may be supplied in either continuous, multi-filament form or in spun form. The denier of these yarns may be between about 20 and 300 denier, with a denier between about 40 and 70 being preferred denier.

The composite yarn can be constructed of a metallic component or a portion thereof. Additionally, the yarn can include multiple components, any of which include metallic material. The metallic component is present in amounts of about 5% to about 100% of the total composite yarn. The ratio can be adjusted to optimize certain mechanical properties to provide the desired end product. For example, the more metallic material present in a composite yarn the more rigid, and non-elastic the composite yarn. This provides a greater mechanical strength yarn having greater burst and tensile strength. Additionally, increased metallic material in the composite yarn provides increased abrasion resistance, and increased radial expansion forces, if used to construct a tubular structure. Alternatively, the lower the percentage of metallic material present in the composite yarn provides for more flexibility and greater elasticity of the composite yarn.

In addition to choosing the materials of construction for the desired end product, the means of constructing the yarn are chosen to provide the desired properties for the end product; i.e. the thickness of the individual components used, the number and direction of twisting the components in forming the yarn, and the number of ply of the yarn. The heaviness, or weight, of the resulting yarn depends on the thickness of the individual components rather than the amount of plies in the yarn. For example, two fat components will result in a yarn much heavier than a 4-ply made of very fine ones. The number of strands, number of twists vary depending on the desired end use and desired properties, such as thickness, strength, flexibility, etc.. For example, more twists which yield a stiffer, stronger yarn are typically desirable for a weaved textile fabric while less twists which yield a softer, flexible yarn is typically desirable for a knitted textile fabric.

The metallic component and the non-metallic component of the present invention are combined using various techniques such as twisting or co-spinning. The twisting or co-spinning technique is performed manually and automatically. There are many different methods to spin fiber into twisted strands, but basically, the prepared material is drawn out and twisted using a spindle or other spinning device, such as a spinning wheel, or the industrial equivalent. The twist lends strength to the strand.

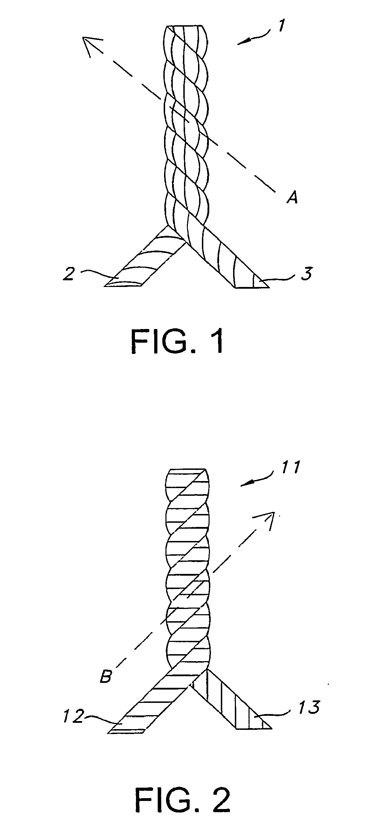

The composite yarn of the present invention is combined by twisting or co-spinning a metallic component with a non-metallic component(s). The twisting or co-spinning can be a "S twist" or "Z twist". The "S twist" is formed by spinning counterclockwise, the resulting twist runs upwards and to the left, as shown in Figure 1 by vector A. The "Z twist" runs opposite to the "S twist". The "Z twist" runs upwards and to the right which is formed by spinning the strand in a clockwise direction, as shown in Figure 2 by vector B. The twist in the strand for Z and S runs in the same direction as the diagonal used to form these letters.

Figure 1 shows each component, metallic component 2 and non-metallic component 3, combined together to form the composite yarn 1. Figure 1 shows a 2-ply composite yarn which includes 1-ply of metallic component 2 twisted together with 1-ply of non-metallic component 3. However, additionally components can be combined or "plied" together to form 3-ply, 4-ply, 5-ply yarns, etc. Plying makes the composite yarn stronger, and more uniform.

Additionally, each component 2, 3 can be pre-twisted prior to combining them to form the composite yarn 1. The components 2, 3 can be pre-twisted as a 1-ply, 2-ply, 3-ply, etc. as well. For example, non-metallic component 3 can include two polymer strands twisted together forming a 2-ply component. The 2-ply component is combined with the metallic component 3 forming the composite yarn of the present invention. The additionally pre-twisted polymer strands provide for more flexibility and dilation, as opposed to the one strand polymer combination.

Figures 1 and 2 shows the composite yarn 1 and 12, respectively, of the present invention in a different twist formation. Figure 1 shows the composite yarn 1 where the metallic component 2 and the non-metallic component 3 are combined using the "S twist". The "S twist" is in the direction of vector A of Figure 1. Figure 2 shows the composite yarn 11 where the metallic component 12 and the non-metallic component 13 are combined using the "Z twist". The "Z twist" is in the direction of vector B of Figure 2. The composite yarns 1 and 11, illustrated in Figure 1 and 2, respectively, may be used alone or combined with other strands to create a variety of composite yarn structures for use in a textile fabric. For example, a composite yarn of the present invention can include various cover yarn(s) and core yarn(s) of which at least one is a metallic component, and one is a non-metallic component. The following examples and figures illustrate variations of the core and cover yarns which can form a composite yarn of the present invention.

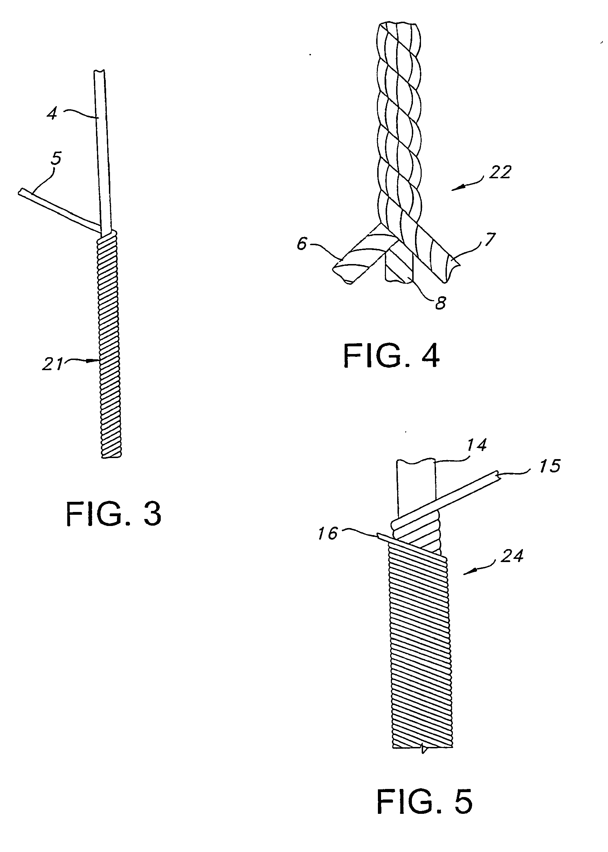

One example illustrated in Figure 3, includes a composite yarn 21 constructed by using a wrapping technique. The wrapping technique is performed using techniques known in the art. The composite yarn 21 includes a core yarn 4 that has been wrapped with a single cover yarn 5. This cover yarn 5 is wrapped about the core yarn 4 at a rate between about 5 and 20 turns per inch. The rate will vary depending on the denier of the core yarn 4 and cover yarn 5 and the material from which they are constructed. Alternatively, Figure 4 shows a composite yarn 22 similar to the composite yarn 21 of Figure 3 but having an additional cover yarn. The composite yarn 22 of Figure 4 includes an additional cover yarn 7 over-wrapped about the core yarn 8 in the same as that of the single cover yarn 6. The cover yarns 6, 7 have a "Z twist" formation about the core yarn 8. The selection of the turns per cm for each of the single yarn 6 and additional cover yarn 7 may be selected using the same criteria described for the composite yarn 21 illustrated in FIG. 3. Additionally, the cover yarns have an "S twist" formation about the core yarn to provide different properties of the composite yarn.

Additionally, Figure 5 illustrates a composite yarn 24 similar to composite yarn 22 of Figure 4 having a core yarn and multiple cover yarns, however, composite yarn 24 has the cover yarns wrapped in opposite direction of each other. The composite yarn 14 includes a first cover yarn 15 which is wrapped in a first direction about the core yarn 14, and a second cover yarn 16 over-wrapped about the cover yarn 15 in the opposite direction to that of the first cover yarn 15. Either of the first cover yarn 15 or second cover yarn 16 may be wrapped at a rate between about 6 to 8 turns per cm with a rate between about 4 and 6 turns per cm being preferred. The number of turns per cm selected for a particular yarn will depend on a variety of factors including, but not limited to, the composition and denier of the yarn, the type of winding equipment that will be used to make the composite yarn, and the desire properties of the prosthesis for the end use.

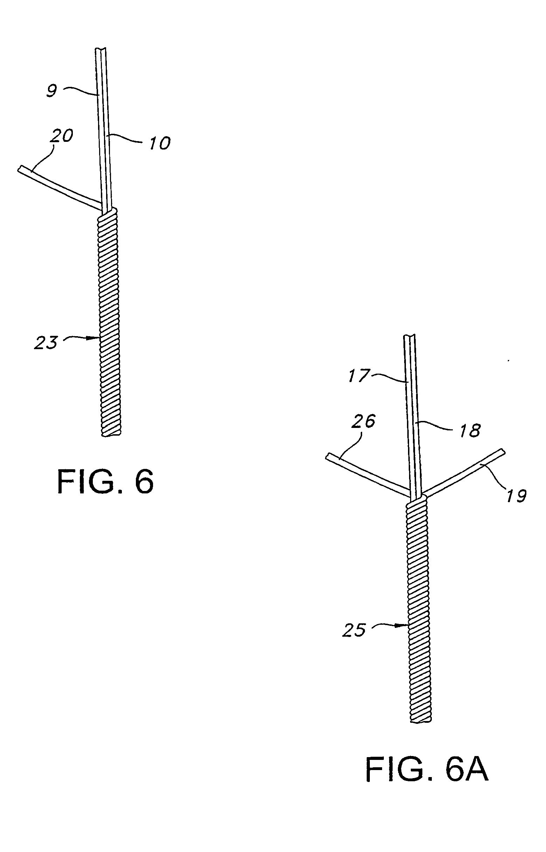

Further examples illustrated in Figures 6 and 6A show cover yarn(s) wrapped around multiple core yarns. Figure 6 shows composite yarn 23 including a first core yarn 9 laid parallel with a second core yarn 10. This two-strand core structure is over-wrapped with a first cover yarn 20 in a first direction, which may be clock-wise or counter clock-wise. Additionally, Figure 6A shows composite yarn 25 having core yarns 17 and 18 wrapped with cover yarns 19 and 26. The core yarns 17 and 18 are similar to that of core yarns 9 and 10 of Figure 6. The cover yarns 19 and 26 of Figure 6A are similar to that of cover yarn 20 of Figure 6. Further, at least one of the core yarns and/or the cover yarn(s) include a metallic component.

A large number of core/cover combinations can be contemplated depending on the yarn available, the characteristics desired in the finished product, and the processing equipment available. For example, any combination of number of strands used, material of construction of the strands, and varying sizes of the strands may be provided for the core yarns and/or the cover yarns. Additionally, any core yarn, cover yarn and combinations thereof may include a metallic component.

Further, various techniques as known in the art may be used to pretreat the materials prior to constructing the composite yarn of the present invention. For example, the non-metallic component may include the step of "drawing" the components. This treatment commonly includes longitudinally stretching the components beyond their point until complete plastic deformation (i.e., a region in which the component now exhibits loss of its elasticity and ability to change appreciably in length) is accomplished.

The force required to "draw" a component increases until the yield point is reached, at which point, the component enters a region of plastic deformation. Once the deformation point in a component has been reached through stretching, the material has substantially lost its elastic memory and is more or less "fixed", neither being able to be further stretched or to return to its original length. Thus, these components retain their expanded circumferential length and now have a fixed diameter.

The composite yarn may also be stretched until a point at which the material fractures, i.e., the fracture point. The process of drawing the yarn to a point prior to the fracture point increases the tensile strength of the yarn and decreases the elongation to failure. As a result of drawing, the polymeric yarns become directionally aligned or oriented.

### INVENTIVE PROSTHESIS

Another embodiment of the present invention is a prosthesis made from a metallic component, metallic yarn, or metallic material. A further embodiment of the present invention is a composite prosthesis including a portion of the prosthesis being constructed of a metallic component, metallic yarn or metallic material. The below discussion pertains to both embodiments and herein referred to as "composite prosthesis" in general.

The yarns used to construct the various composite prostheses of the present invention, generally, can be flat, twisted, textured or combinations thereof, and may have high, low or moderate shrinkage properties. Additionally, the yarn type and yarn denier can be selected to meet specific properties desired for the prosthesis, such as porosity, flexibility and compliance, as above-discussed. The yarn denier represents the linear density of the yarn. Thus, yarn of small denier, e.g., 40-50 denier, would correspond to a very fine yarn whereas a yarn with a larger denier, e.g. 1,000, would correspond to a heavy yarn.

Yarns useful in the inventive composite prostheses have a denier range from about 20 to about 1500 and a filament count of about 10 to about 200, depending on the specific type of prosthesis and the use of the desired product. The yarns used with the present invention preferably have a denier from about 20 to about 300. A high filament count for the same overall linear density increases the yarns flexibility, reduces its stiffness and reduces permeability to viscous liquids, i.e. blood.

Further, the composite prosthesis of the present invention includes a yarn as above-discussed having at least a portion being metallic. The flexibility, durability, stiffness and strength can be adjusted by varying the percent ratio of metallic component in the composite prosthesis, as above-discussed.

The composite prosthesis of the present invention can have virtually any textile construction, including weaves, knits, braids, filament windings and the like.

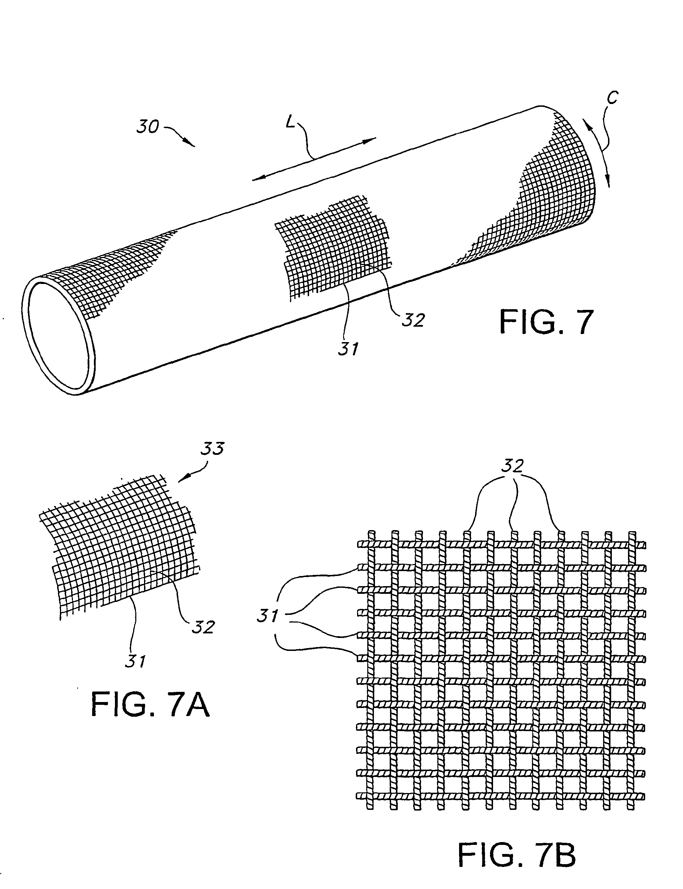

A variety of textile constructions may be employed using the present invention. With respect to weaves, any known weave pattern in the art, including, simple weaves, basket weaves, twill weaves, velour weaves and the like may be used. Referring to the drawings and, in particular to Figure 7 showing a woven tubular prosthesis 30, Figure 7A showing a woven patch prosthesis 33, and Figure 7B showing the enlarged weave of the prostheses. Figures 7 and 7A show that a weave pattern includes warp yarns 31 and fill yarns 32, the fill yarns being at approximately 90 degrees to one another with composite fabric flowing from the machine in the warp direction. For example, as shown in Figure 7, the weave pattern includes warp yarns 31 running along the longitudinal length (L) of the woven product and fill yarns 32 running around the circumference (C) of the product. As discussed prior, this treatment commonly includes the step of "drawing" the yarns, that is, longitudinally stretching the yarns beyond their point until complete plastic deformation is accomplished (i.e., a region in which the yarn now exhibits loss of its elasticity and ability to change appreciably in length).

A composite prosthesis that is woven with undrawn or a combination of undrawn and partially drawn radial yarns is also contemplated. Such composite prostheses will be capable of circumferential expansion following manufacture of the product. For instance, if a balloon catheter (or similar device) is inserted into such a composite prosthesis and is thereafter expanded, the composite prosthesis will circumferentially expand a slight degree until the yield point is reached. At that point, the radial yarns, i.e. fill yarns, which were not drawn, will plastically deform, thereby allowing substantial circumferential expansion.

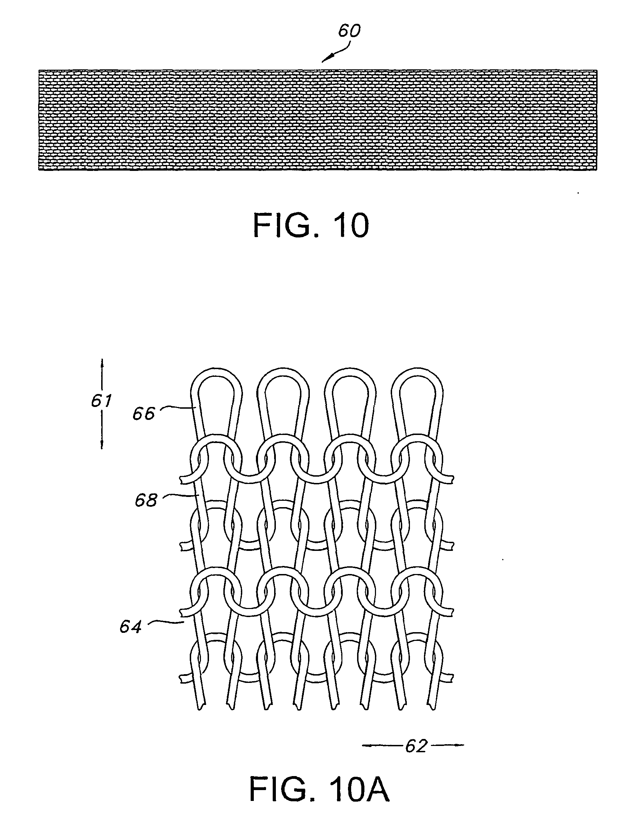

In addition to woven textile composite prostheses, knitted textile composite prostheses are provided. Knitting involves the interlooping or stitching of yarn into vertical columns (wales) and horizontal rows (courses) of loops to form the knitted fabric structure. Warp knitting is particularly useful with the knitted textile portions of the present invention. In warp knitting, the loops are formed along the textile length, i.e., in the wale or warp direction of the textile. As depicted in FIG. 10 and 10A, a composite prosthesis 60, stitches in the axial or longitudinal direction of the textile are called wales (indicated by vector 61) and stitches in the latitudinal, radial or circumferential direction of the textile are called courses (indicated by vector 62). Yarns 66 and 68 interloop in the warp direction to form a warp-knitted 64.

Knitting patterns useful with the present invention include conventional warp-knitted patterns and high-stretch, warp-knitted patterns. Commonly used warp-knitted patterns include locknit (also referred to as tricot or jersey knits), reverse locknit, sharkskin, queenscord and velour knits. Useful high stretch, warp-knitted patterns include those with multiple patterns of diagonally shifting yarns, such as certain modified atlas knits which are described in U.S. Patent No. 6,540,773 . Other useful high-stretch, warp knitted patterns include certain patterns with multiple needle underlap and one needle overlap, such as those patterns described in U.S. Patent No. 6,554,855 and U.S. Patent Application Publication No. 2003/0204241 A1.

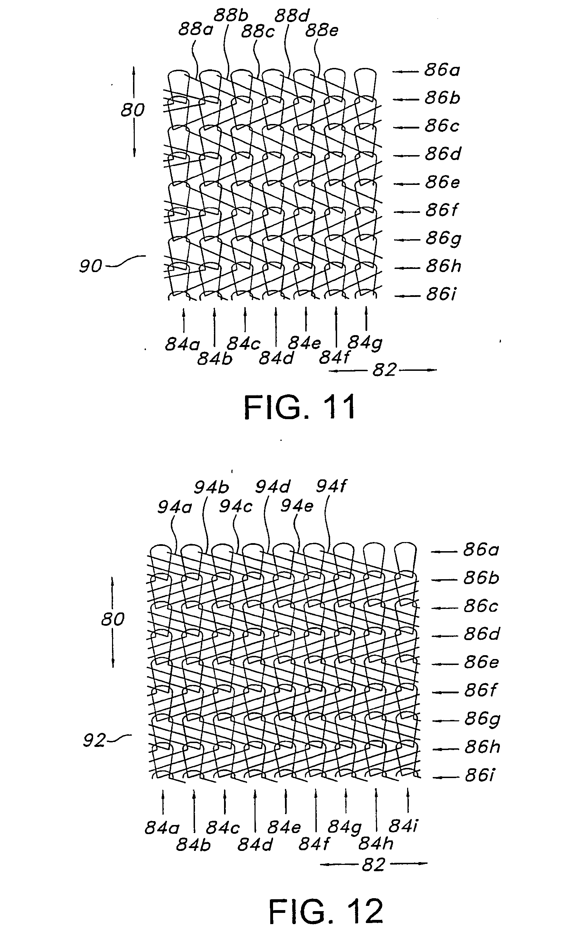

FIG. 11 is an illustration of a high-stretch knitted pattern 90 useful with the present invention having a two needle underlap. In FIG. 11, needle positions in the course direction, i.e., vector 82, are noted by element numbers 84a through 84g and needle positions in the wale direction, i.e., vector 80, are noted by element numbers 86a through 86i. Yarn 88a travels in the course direction from needle position 84a to needle position 84c, or two needle positions, before interlooping with yarn 88c. Yarn 88a then travels two needle positions in the opposite course direction to interloop with a yarn. This alternating two needle position movement is repeated with different yarns to form the knitted pattern 90 with a two needle underlap.

The knitted portion 92, as illustrated in FIG. 12, is characterized as a three-needle underlap. In FIG. 12, needle positions in the course direction, i.e., vector 82, are noted by element numbers 84a through 84i and needle positions in the wale direction, i.e., vector 80, are noted by element numbers 86a through 86i. Yarn 94a travels in the course direction from needle position 84a to needle position 84d, or three needle positions, before interlooping with yard 94d. Yarn 94a then travels three needle positions in the opposite course direction to interloop with a yarn. This alternating three needle position movement is repeated with different yarns to form the knitted pattern 92 with a three needle underlap.

The knitted patterns 64, 90 and 92 are depicted as a single knitted layer in FIGS. 10-12 . The textile portions of the present invention, however, are not so limited. For instance, the knitted portions may also include more than one layer of interconnected yarns. In such a multi-layered knitted textile, yarns from one layer are often interlooped with yarns in another layer to form the multi-layered knitted textile.

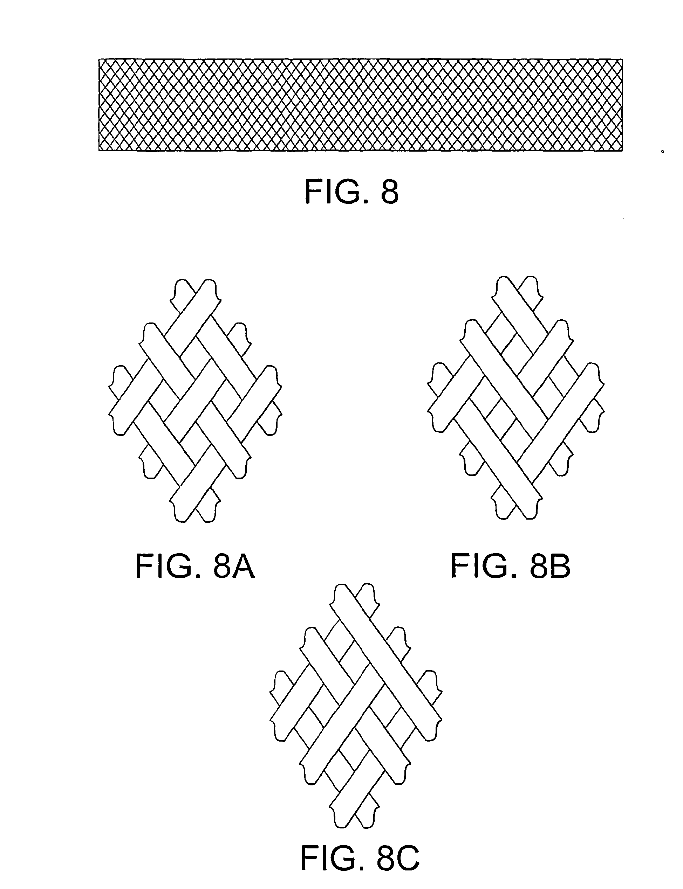

Although weaving and knitting are among the most desirable constructions, braiding may also be used as shown in Figures 8 and 8A. Braiding of yarns includes the interlacing of two yarn systems such that the path of the yarns are diagonal to the fabric delivery direction, forming either a flat or tubular structure. A multi-layered braided structure is defined as a structure formed by braiding wherein the structure has a plurality of distinct and discrete layers. These layers may be bound by interlocking yarns or by adhesive laminates, sewing or the like.

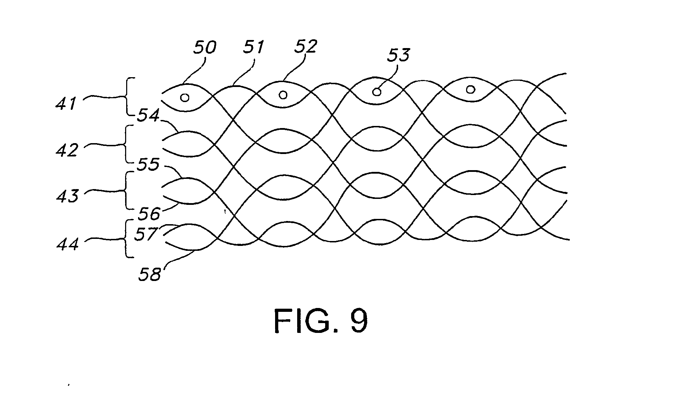

An interlocking three-dimensional braid, as shown in Figure 9, may be used and is defined as a braided structure having at least two layers, whereby a yarn is interbraided from a first layer into a contiguous second layer to interlock the layers of the multi-layer braid. Referring to Figure 9, the composite prosthesis of the present invention includes four layers, 41, 42, 43 and 44, with each layer having at least one interlocking yarn from a contiguous layer. The interlocking yarns are braided into the structure so that the yarn forms part of the first layer, as well as being part of the contiguous layer by forming the interlock. Within each layer, a segment of the braid is formed by an interlocking yarn from a contiguous layer, the layers being interbraided together. The interlocking yarns couple the multiple layers together to form a three-dimensional braid.

In Figure 9, the first layer 41 forms the outer layer of the interlocking three-dimensional braided structure. The outer layer is formed from a yarn 51 which is exclusively braided into the first layer along with a yarn 50 which is interbraided into a second layer which is contiguous with the first layer and a yarn 52 which is interbraided from the second layer up into the first layer. The second layer 42 is formed from segments of four yarns 50, 52, 54 and 56 which are interbraided.

The next contiguous layer 43 is formed from segments of four yarns 54, 55, 56 and 58 inter-braided to form an inner layer in the multilayered structure. Layer 44 is formed in similar fashion, having three yarns 55, 57 and 58 which are interbraided.

A solid three-dimensional braided structure, may be used and is formed by continuous intertwining of the fibers. Solid three-dimensional braids are homogenous in that all yarns are present throughout the thickness of the braid. Typically, three-dimensional braiding machines used to form this type of solid braid include an array of fiber bobbins held in ring or track configurations. Circumferential motion of the array of the bobbins to form the braid is accomplished by shifting slotted rings containing the fiber holders. Fibers are directed through the thickness of the braid by shifting the holders between the rings. Reversal of the direction of ring and hold motions during the shift segment interlocks the fibers. Since every fiber undergoes a similar motion, all fibers become entwined in the balanced array.

Further, this invention contemplates a method for making a biocompatible, implantable prosthesis including the steps of providing a metallic component. Forming a metallic yarn.. Selecting a textile construction and forming a prosthesis in accordance with the selected textile pattern. Various textile constructions may be used such as weaving, knitting, braiding, non-woven spinning and combinations thereof to construct a prosthesis having the desired properties.

Furthermore, this invention contemplates a method for making a biocompatible, implantable prosthesis including the steps of providing a composite yarn which includes a combination of a metallic component and non-metallic component. The combination of the components includes the steps of co-spinning, twisting, and combinations thereof Additional steps of this method include selecting a textile construction and forming a prosthesis in accordance with the selected textile pattern. Various textile constructions may be used such as weaving, knitting, braiding, non-woven spinning and combinations thereof to construct a prosthesis having the desired properties.

Virtually any type of implantable prosthesis can be made from the present invention due to the versatility of the metallic component of the present invention or composite yarn of the present invention. Particularly desirable applications include intraluminal prostheses, such as endovascular grafts, blood filters, stent-graft composites, balloon catheter, filter, mesh, valves, vascular patch, hernia plug, arterial-vascular access graft and the like. This invention may also be designed to repair or support a weakened or damaged lumen, such as a blood vessel in the vascular system.

Although the present invention has been described with preferred embodiments, it is to be understood that modifications and variations may be utilized within the purview and scope of the appended claims and their equivalents.

## Claims

1. An implantable prosthesis (30) comprising a biocompatible implantable fabric having a textile construction comprising a composite yarn (1), said composite yarn (1) comprising a combination of a metallic component (2) and a non-metallic component (3), **characterized in that**, said combination is selected from a group consisting of twisting, co-spinning, and combination thereof.

2. The implantable prosthesis (30) of claim 1 wherein said combination is an "S-twist" formation of said non-metallic component(3) and said metallic component (2).

3. The implantable prosthesis (30) of claim 2 further comprising a second non-metallic component wrapped about said composite yarn (1) in a "Z-twist" direction.

4. The implantable prosthesis (30) according to claim 2, wherein said non-metallic component (3) is pre-twisted in an "Z-twist" formation prior to said "S-twist" formation with said metallic component (2).

5. The implantable prosthesis (30) of claim 1 wherein said composite yarn (1) comprises a "Z-twist" of said non-metallic component (3) and said metallic component (2).

6. The implantable prosthesis (30) of claim 5 further comprising a second non-metallic component wrapped about said composite yarn (1) in an "S-twist" direction.

7. The implantable prosthesis (30) according to claim 5, wherein said non-metallic component is pre-twisted in an "S-twist" formation prior to said "Z-twist" formation with said metallic component (2).

8. The implantable prosthesis (30) of claim 1 wherein said composite yarn (1) has a ratio of non-metallic component (3) to metallic component (2) between about 5% to 95%.

9. The implantable prosthesis (30) of claim 1 wherein said metallic component (2) is selected from the group consisting of stainless steel, titanium, gold, nitinol, and combinations and derivatives thereof.

10. The implantable prosthesis (30) of claim 9, wherein said metallic component (2) has a diameter of about 0.05 mm to about 0.5 mm.

11. The implantable prosthesis (30) of claim 1 wherein said non-metallic component (3) is selected from the group consisting of polymeric filaments, PET, PTFE, polypropylene, and combinations and derivatives thereof.

12. The implantable prosthesis (30) according to claim 11, wherein said non-metallic component (3) has a denier from about 20 to about 150.

13. The implantable prosthesis (30) according to claim 1 wherein said textile construction is selected from the group consisting of weaves, knits, braids, windings, spinnings and combinations thereof

14. The implantable prosthesis (30) according to claim 1 wherein said implantable prosthesis (30) is an endovascular graft.

15. The implantable prosthesis (30) according to claim 1 wherein said implantable prosthesis (30) is selected from the group consisting of a balloon catheter, filter, mesh, valves, vascular patch, hernia plug and arterial-vascular access graft.

16. The implantable prosthesis (30) according to claim 1, further comprising a cover fiber wrapped about said composite yarn (1).

17. Method for making a implantable prosthesis (30) comprising:
a) providing a composite yarn (1) comprising a combination of a metallic component (2) and a non-metallic component (3), said combination is selected from a group consisting of twisting, co-spinning, and combination thereof; and
b) forming the composite yarn (1) into said implantable prosthesis (30) by using a textile construction selected from a group consisting of weaving, knitting, braiding, non-woven spinning and combinations thereof.

18. The method according to claim 17, wherein said step of providing further includes twisting said non-metallic component (3) and said metallic component (2) in an "S-twist" direction.

19. The method according to claim 17, wherein said step of providing further includes twisting said non-metallic component (3) and said metallic component (2) in an "Z-twist" direction.

## Patentansprüche

1. Implantierbare Prothese (30), die aufweist: ein biokompatibles implantierbares Textilerzeugnis mit einer Textilkonstruktion, die ein Verbundgarn (1) aufweist, wobei das Verbundgarn (1) eine Kombination aus einer metallischen Komponente (2) und einer nichtmetallischen Komponente (3) aufweist, **dadurch gekennzeichnet, daß** die Kombination aus einer Gruppe ausgewählt ist, die aus Verdrehen, gemeinsamem Verspinnen und deren Kombination besteht.

2. Implantierbare Prothese (30) nach Anspruch 1, wobei die Kombination eine "S-Drehungs-"Bildung aus der nichtmetallischen Komponente (3) und der metallischen Komponente (2) ist.

3. Implantierbare Prothese (30) nach Anspruch 2, ferner mit einer zweiten nichtmetallischen Komponente, die um das Verbundgarn (1) in "Z-Drehungs-"Richtung gewickelt ist.

4. Implantierbare Prothese (30) nach Anspruch 2, wobei die nichtmetallische Komponente (3) vor der "S-Drehungs-"Bildung mit der metallischen Komponente (2) in einer "Z-Drehungs-"Bildung vorgedreht ist.

5. Implantierbare Prothese (30) nach Anspruch 1, wobei das Verbundgarn (1) eine "Z-Drehung" der nichtmetallischen Komponente (3) und der metallischen Komponente (2) aufweist.

6. Implantierbare Prothese (30) nach Anspruch 5, ferner mit einer zweiten nichtmetallischen Komponente, die um das Verbundgarn (1) in "S-Drehungs-"Richtung gewickelt ist.

7. Implantierbare Prothese (30) nach Anspruch 5, wobei die nichtmetallische Komponente vor der "Z-Drehungs-"Bildung mit der metallischen Komponente (2) in einer "S-Drehungs-"Bildung vorgedreht ist.

8. Implantierbare Prothese (30) nach Anspruch 1, wobei das Verbundgarn (1) ein Verhältnis von nichtmetallischer Komponente (3) zu metallischer Komponente (2) zwischen etwa 5 % bis 90 % hat.

9. Implantierbare Prothese (30) nach Anspruch 1, wobei die metallische Komponente (2) aus der Gruppe ausgewählt ist, die aus Edelstahl, Titan, Gold, Nitinol sowie deren Kombinationen und Abkömmlingen besteht.

10. Implantierbare Prothese (30) nach Anspruch 9, wobei die metallische Komponente (2) einen Durchmesser von etwa 0,05 mm bis etwa 0,5 mm hat.

11. Implantierbare Prothese (30) nach Anspruch 1, wobei die nichtmetallische Komponente (3) aus der Gruppe ausgewählt ist, die aus Polymerfäden, PET, PTFE, Polypropylen sowie deren Kombinationen und Abkömmlingen besteht.

12. Implantierbare Prothese (30) nach Anspruch 11, wobei die nichtmetallische Komponente (3) ein Denier von etwa 20 bis etwa 150 hat.

13. Implantierbare Prothese (30) nach Anspruch 1, wobei die
Textilkonstruktion aus der Gruppe ausgewählt ist, die aus Geweben, Gewirken bzw. Gestricken, Geflechten, gewickelten Gebilden, Gespinsten und deren Kombinationen besteht.

14. Implantierbare Prothese (30) nach Anspruch 1, wobei die implantierbare Prothese (30) ein endovaskuläres Implantat ist.

15. Implantierbare Prothese (30) nach Anspruch 1, wobei die implantierbare Prothese (30) aus der Gruppe ausgewählt ist, die aus einem Ballonkatheter, einem Filter, Maschenmaterial, Klappen, einem Gefäßpatch, einer Hernienplombe und einem arteriell-vaskulären Zugangsimplantat besteht.

16. Implantierbare Prothese (30) nach Anspruch 1, ferner mit einer Abdeckfaser, die um das Verbundgarn (1) gewickelt ist.

17. Verfahren zur Herstellung einer implantierbaren Prothese (30) mit:
a) Bereitstellen eines Verbundgarns (1), das eine Kombination aus einer metallischen Komponente (2) und einer nichtmetallischen Komponente (3) aufweist, wobei die Kombination aus einer Gruppe ausgewählt ist, die aus Verdrehen, gemeinsamem Verspinnen und deren Kombination besteht; und
b) Umformen des Verbundgarns(1) in die implantierbare Prothese durch Verwenden einer Textilkonstruktion aus einer Gruppe, die aus Weben, Wirken bzw. Stricken, Flechten, Vliesspinnen und deren Kombinationen besteht.

18. Verfahren nach Anspruch 17, wobei der Schritt des Bereitstellens ferner aufweist: Drehen der nichtmetallischen Komponente (3) und der metallischen Komponente (2) in "S-Drehungs-"Richtung.

19. Verfahren nach Anspruch 17, wobei der Schritt des Bereitstellens ferner aufweist: Drehen der nichtmetallischen Komponente (3) und der metallischen Komponente (2) in "Z-Drehungs-"Richtung.

## Revendications

1. Prothèse implantable (30) comprenant un tissu implantable biocompatible ayant une construction textile comprenant un fil composite (1), ledit fil composite (1) comprenant une combinaison d'un élément métallique (2) et d'un élément non métallique (3), **caractérisée en ce que** ladite combinaison est choisie dans le groupe constitué par une torsion, un cofilage et une combinaison de ceux-ci.

2. Prothèse implantable (30) selon la revendication 1, dans laquelle ladite combinaison est une formation de "torsion en S" dudit élément non métallique (3) et dudit élément métallique (2).

3. Prothèse implantable (30) selon la revendication 2, comprenant en outre un second élément non métallique enroulé autour dudit fil composite (1) dans une direction de "torsion en Z".

4. Prothèse implantable (30) selon la revendication 2, dans laquelle ledit élément non métallique (3) est pré-torsadé dans une formation de "torsion en Z" avant ladite formation de "torsion en S" avec ledit élément métallique (2).

5. Prothèse implantable (30) selon la revendication 1, dans laquelle ledit fil composite (1) comprend une "torsion en Z" dudit élément non métallique (3) et dudit élément métallique (2).

6. Prothèse implantable (30) selon la revendication 5, comprenant en outre un second élément non métallique enroulé autour dudit fil composite (1) dans une direction de "torsion en S".

7. Prothèse implantable (30) selon la revendication 5, dans laquelle ledit élément non métallique est pré-torsadé dans une formation de "torsion en S" avant ladite formation de "torsion en Z" avec ledit élément métallique (2).

8. Prothèse implantable (30) selon la revendication 1, dans laquelle ledit fil composite (1) a un rapport élément non métallique (3) sur élément métallique (2) entre environ 5 % et 95 %.

9. Prothèse implantable (30) selon la revendication 1, dans laquelle ledit élément métallique (2) est choisi dans le groupe constitué par l'acier inoxydable, le titane, l'or, le nitinol et des combinaisons et dérivés de ceux-ci.

10. Prothèse implantable (30) selon la revendication 9, dans laquelle ledit élément métallique (2) a un diamètre d'environ 0,05 mm à environ 0,5 mm.

11. Prothèse implantable (30) selon la revendication 1, dans laquelle ledit élément non métallique (3) est choisi dans le groupe constitué par les filaments polymères, le PET, le PTFE, le polypropylène et des combinaisons et dérivés de ceux-ci.

12. Prothèse implantable (30) selon la revendication 11, dans laquelle ledit élément non métallique (3) a un denier d'environ 20 à environ 150.

13. Prothèse implantable (30) selon la revendication 1, dans laquelle ladite construction textile est choisie dans le groupe constitué par les tissés, tricots, tresses, enroulements, filages et combinaisons de ceux-ci.

14. Prothèse implantable (30) selon la revendication 1, dans laquelle ladite prothèse implantable (30) est une greffe endovasculaire.

15. Prothèse implantable (30) selon la revendication 1, dans laquelle ladite prothèse implantable (30) est choisie dans le groupe constitué par un cathéter à ballonnet, un filtre, une maille, des valves, un patch vasculaire, un bouchon d'hernie et une greffe d'accès artérielle-vasculaire.

16. Prothèse implantable (30) selon la revendication 1, comprenant en outre une fibre de couverture enroulée autour dudit fil composite (1).

17. Procédé de fabrication d'une prothèse implantable (30) comprenant :
a) fournir un fil composite (1) comprenant une combinaison d'un élément métallique (2) et d'un élément non métallique (3), ladite combinaison étant choisie parmi un groupe constitué par une torsion, un cofilage et une combinaison de ceux-ci ; et
b) former le fil composite (1) en ladite prothèse implantable (30) en utilisant une construction textile choisi à partir d'un groupe constitué par le tissage, le tricotage, le tressage, le filage non tissé et des combinaisons de ceux-ci.

18. Procédé selon la revendication 17, dans lequel ladite étape de fourniture comprend en outre : torsader ledit élément non métallique (3) et ledit élément métallique (2) dans une direction de "torsion en S".

19. Procédé selon la revendication 17, dans lequel ladite étape de fourniture comprend en outre : torsader ledit élément non métallique (3) et ledit élément métallique (2) dans une direction de "torsion en Z".
